# FASCICULE DE BREVET EUROPEEN

(11) **EP 3 644 056 B1**
(45) Date de publication et mention de la délivrance du brevet: **28.08.2024**
(21) Numéro de dépôt: 19205462.5
(22) Date de dépôt: 25.10.2019
(51) Int. Cl.: G01N 33/00, G01N 33/22, G01N 21/3504, G01N 30/02

(54) **DISPOSITIF D'ANALYSE DE GAZ ET PROCEDE D'ANALYSE**
GASANALYSEVORRICHTUNG UND ANALYSEVERFAHREN
DEVICE FOR ANALYSING GAS AND METHOD OF ANALYSIS

(30) Priorité: 26.10.2018 FR 1859957
(43) Date de publication de la demande: 29.04.2020
(73) Titulaire: Eiffage Energie Systemes - Participations, 78140 Velizy-Villacoublay (FR)
(72) Inventeur: FREDERIC, Xavier, 78140 VELIZY-VILLACOUBLAY (FR)
(74) Mandataire: Cabinet Netter

(56) Documents cités:
- FR-A1- 3 064 718
- US-A1- 2007 273 882

## Description

### Domaine technique

La présente invention se rapporte au domaine technique général de l'analyse de gaz et notamment de l'analyse de gaz de combustion. Ces gaz comprennent notamment le gaz naturel, les biogaz et le biométhane.

Il est en effet nécessaire d'analyser les différents constituants d'un biogaz ou biométhane avant son injection dans un réseau de distribution ou de transport. La présence de certains composés, même en faibles quantités ou sous forme de traces, peut être nocive pour les réseaux, comme le sont par exemple les composés souffrés.

Il est ainsi impératif d'effectuer des vérifications concernant un certain nombre de composés, comme le taux d'H₂S, lequel est particulièrement corrosif, dans un biogaz avant son injection dans un réseau. En fonction de la valeur de ce taux, il peut être nécessaire de traiter le biogaz avant sont injection dans un réseau.

L'apparition d'un nombre de plus en plus important de producteurs de biogaz impose par conséquent un contrôle de qualité des biogaz / biométhane et le cas échéant, également une adaptation des matériels et dispositifs existants aux propriétés de des biogaz.

La présente invention concerne donc plus particulièrement l'analyse des constituants de biogaz / biométhane.

### Etat de la technique

Pour analyser des gaz destinés à être injectés dans une canalisation de distribution ou de transport, il est connu d'utiliser des postes d'analyse de gaz équipés de plusieurs appareils distincts.

A titre d'exemple, un poste d'analyse pour analyser du biométhane comprend un ensemble d'appareils à savoir:
- un analyseur transactionnel pour mesurer le pouvoir calorifique supérieur du gaz (PCS),
- un analyseur H₂O, pour mesurer le taux d'H₂O dans le gaz,
- un analyseur de sulfure d'hydrogène et d'oxyde de carbone (H₂S/COS)
- un analyseur de Wobbe,
- un appareil de mesure du Tétrahydrothiophène (THT),
- un analyseur de méthane, azote, oxygène et dioxyde de carbone.

Chaque appareil nécessite une boucle d'échantillonnage spécifique, augmentant ainsi la consommation de gaz à échantillonner. En outre, les modalités de mise en forme du gaz (pression, température, débit, filtration, évents, gaz vecteur) pour l'échantillonnage sont différentes d'un appareil à l'autre, augmentant la complexité et les coûts des opérations d'analyse. Il faut en effet, généralement utiliser des filtres spécifiques en fonction des caractéristiques des analyseurs, nécessitant par ailleurs une maintenance périodique.

Les appareils connus fonctionnent avec une circulation continue du gaz à analyser et par conséquent avec un rejet d'une quantité importante de gaz dans l'atmosphère.

Les différents évents utilisés pour les mesures favorisent un cumul de gaz au niveau desdits évents et entraînent par conséquent un rejet important de gaz dans l'atmosphère.

En outre, il est connu de l'US 2007/273882 A1 d'arranger un spectromètre laser en aval d'un chromatographe en phase gazeuse pour analyser des gaz de combustion.

### Exposé de l'invention

L'objet de la présente invention vise par conséquent à pallier les inconvénients de l'art antérieur et de fournir un nouveau dispositif d'analyse de gaz de combustion permettant de déceler davantage de composés dudit gaz de combustion.

Un autre objet de l'invention vise à réduire substantiellement les quantités de gaz nécessaires pour l'analyse et par conséquent rejetées dans l'atmosphère.

Un autre objet de la présente invention vise à fournir un nouveau dispositif d'analyse de gaz de combustion dont l'utilisation est simple et fiable.

Un autre objet de la présente invention vise à fournir un nouveau dispositif d'analyse de gaz de combustion permettant de diminuer substantiellement les coûts liés à l'utilisation dudit dispositif.

Un autre objet de la présente invention vise à proposer un nouveau procédé d'analyse basé sur l'utilisation du dispositif conforme à l'invention.

Un autre objet de la présente invention vise à proposer un nouveau programme d'ordinateur pour mettre en oeuvre le procédé conforme à l'invention.

Les objets assignés à l'invention sont atteints à l'aide d'un dispositif d'analyse de gaz de combustion comprenant :
- une unité de micro-chromatographie en phase gazeuse pour mesurer la concentration de composés du gaz de combustion,
- une unité d'échantillonnage et de calibration pour prélever les échantillons à analyser dans le gaz de combustion, et
- une interface utilisateur pour lancer et contrôler le déroulement de l'opération d'analyse et pour visionner les résultats de l'analyse à l'aide d'un outil d'affichage, caractérisé en ce qu'il comprend une unité laser comportant au moins un module laser, raccordée par une liaison pneumatique en série et en amont à l'unité de micro-chromatographie pour mesurer la concentration de composés additionnels du gaz de combustion, une unité de traitement et de contrôle automatisée pour l'acquisition et le traitement des mesures et pour effectuer des calculs nécessaires à partir des valeurs mesurées, l'unité laser et l'unité de micro-chromatographie effectuant les analyses respectives et successives sur les mêmes échantillons fournis grâce à l'unité d'échantillonnage et de calibrage commune.

Selon un exemple de réalisation du dispositif d'analyse, l'unité d'échantillonnage et de calibrage comprend une tubulure d'analyse équipée d'une vanne d'admission pilotée, ladite tubulure d'analyse raccordant en série l'unité laser et l'unité de micro-chromatographie pour faire circuler une fraction déterminée du gaz à analyser successivement dans l'unité laser puis dans l'unité de micro-chromatographie.

Selon un exemple de réalisation, le dispositif d'analyse comprend au moins un système de prélèvement par aspiration raccordé à la tubulure d'analyse.

Selon un exemple de réalisation du dispositif d'analyse, la tubulure d'analyse présente un diamètre inférieur ou égal à 3,2 mm.

Selon un exemple de réalisation, le dispositif d'analyse comprend un système de communication pour l'acquisition, la mise à disposition et la transmission de signaux mesurés et calculés et/ou de données.

Selon un exemple de réalisation du dispositif d'analyse, le système de communication comprend des outils de réception et de transmission de données, configurés pour constituer un dispositif d'analyse connecté.

Selon un exemple de réalisation, le dispositif d'analyse comprend une unité infra-rouge pour effectuer des mesures additionnelles sur le gaz circulant dans la tubulure d'analyse.

Selon un exemple de réalisation du dispositif d'analyse, l'unité de traitement et de contrôle automatisée comprend des cartes de communication Ethernet, une carte CPU, des cartes entrées / sorties et un afficheur tactile.

Les objets assignés à l'invention sont atteints également à l'aide d'un procédé d'analyse de gaz de combustion au préalable de son injection dans une canalisation ou dans un réseau de distribution, mis en oeuvre en utilisant un dispositif d'analyse présenté ci-dessus, caractérisé en ce qu'il comprend les étapes suivantes:
- ouvrir une vanne d'admission ou d'analyse pendant une durée déterminée pour faire circuler le gaz à analyser dans la tubulure d'analyse,
- prélever un échantillon de gaz grâce au système de prélèvement par aspiration ou par circulation naturelle dans une conduite,
- soumettre l'échantillon à l'unité laser,
- soumettre l'échantillon à l'unité de micro-chromatographie,
- effectuer un traitement et un contrôle des valeurs mesurées de manière à fournir des résultats fiables et exploitables, et
- répéter les étapes ci-dessus avec une périodicité déterminée.

Selon un exemple de mise en oeuvre du procédé d'analyse, la période de mise en oeuvre des étapes dudit procédé est comprise entre 70 secondes et 75 secondes pour du biométhane, de manière à fournir un résultat d'analyse actualisé périodiquement.

A titre de comparaison, avec les systèmes connus, cette durée ou période est environ de 120 secondes pour le biométhane.

Un tel gain de temps permet de simplifier l'installation, notamment sur un plan dimensionnel, car pour pouvoir injecter un gaz dans une canalisation, il faut vérifier sa qualité. Plus le temps d'analyse est long, plus le gaz doit être temporisé dans l'attente du résultat. Temporiser le gaz signifie que le détenteur de l'installation utilise une capacité qui est proportionnée en fonction de volume nécessaire à temporiser. Ce volume nécessaire pour stocker le gaz est directement lié au temps d'analyse. Ainsi, plus le temps d'analyse est long, plus le volume de gaz à temporiser doit être grand, augmentant par conséquent le coût de l'installation.

Selon un exemple de mise en oeuvre, le procédé d'analyse consiste, à l'aide de l'unité de traitement et de contrôle, de réajuster les valeurs mesurées par l'unité laser en utilisant les valeurs mesurées par l'unité de micro-chromatographie.

Le procédé d'analyse permet, grâce à un logiciel ou programme d'ordinateur spécifique, exécuté sur un automate ou sur un ordinateur, de collecter des mesures issues des unités de micro-chromatographie, laser, ou infrarouge, de corriger ou d'ajuster lesdites mesures, de les convertir (changement d'unités, changement de conditions de référence etc...) et de surveiller des paramètres (par exemple un seuil d'alarme).

Ainsi, le procédé d'analyse permet par exemple de calculer le pouvoir calorifique du gaz à analyser, sa masse volumique, la vitesse du son du gaz et des moyennes de divers paramètres.

A titre d'exemple, le pouvoir calorifique du gaz à analyser est calculé en fonction de ses différents composants et comme le procédé d'analyse conforme à l'invention permet d'analyser un plus grand nombre de composants, les résultats obtenus du calcul du pouvoir calorifique sont plus précis.

Le dispositif d'analyse, intègre ainsi, selon l'invention, une technologie hybride permettant de détecter plus rapidement que les dispositifs connus, la présence d'un composant ou la modification d'un composant du gaz à analyser. Les composants polluants ou nocifs sont ainsi détectés plus rapidement.

Selon un exemple de mise en oeuvre, le procédé d'analyse consiste à sélectionner, parmi une liste de composants du gaz à analyser, une partie au moins desdits composants susceptibles d'être détectés, lesquels seront analysés. Une telle sélection peut dépendre de la nature et/ou de l'origine du gaz.

Les objets assignés à l'invention sont également atteints à l'aide d'un produit programme d'ordinateur comprenant des instructions de code de programme enregistrées sur un support lisible par ordinateur pour mettre en oeuvre les étapes du procédé présenté ci-dessus, lorsque ledit programme fonctionne sur un ordinateur.

L'invention procure un avantage considérable dans la mesure où elle permet de diminuer substantiellement les quantités de gaz nécessaires aux analyses et par conséquent les quantités de gaz rejetées dans l'atmosphère, grâce à la mutualisation du gaz lors de l'échantillonnage commun à toutes les unités.

Les coûts liés à l'utilisation d'un tel dispositif d'analyse sont par conséquent diminués.

Un autre avantage de l'invention réside dans le nombre accru de composés, décelés dans des gaz de combustion et ce à l'aide un dispositif d'analyse simple, fiable et peu encombrant.

Un autre avantage de l'invention réside dans le fait que le dispositif d'analyse remplace quatre analyseurs spécifiques indépendants actuels.

Les rejets dans l'atmosphère sont avantageusement limités à 1-2 ml/min contre 600 ml/min pour l'ensemble des analyseurs actuels.

Le dispositif conforme à l'invention fonctionne avantageusement par intermittence et ne nécessite pas de débit continu du gaz à analyser.

En outre, la consommation de gaz vecteur dans l'unité de micro-chromatographie est limitée à 2-12 ml/min contre 72 ml/min, environ, pour les systèmes connus.

A titre d'exemple, le dispositif d'analyse conforme à l'invention, combinant des technologies différentes, permet de réduire la consommation d'hélium laquelle est de 2,4 ml/min par module contre 8,5 à 17 ml/min pour les systèmes connus. Ceci est lié au fait que le micro-chromatographe ne consomme que peu de gaz vecteur, notamment grâce au fonctionnement par intermittence et que l'unité laser n'utilise pas de gaz vecteur.

En outre, l'échantillonnage du gaz à analyser est commun pour toutes les unités de mesure, permettant ainsi de simplifier les opérations de maintenance.

Avantageusement, les fonctionnements respectifs de l'unité de micro-chromatographie et de l'unité laser ne sont pas modifiés du fait de l'intégration dans un seul et même dispositif conforme à l'invention. Ce dernier permet cependant d'effectuer des corrections ou ajustages, le cas échéant d'améliorer la précision, d'effectuer des calculs, des changements d'unité ou d'autres traitements de valeurs mesurées ou de données. Le dispositif conforme à l'invention permet ainsi d'optimiser et d'homogénéiser les résultats d'analyses, leur présentation et/ou affichage.

Pour la mesure de la concentration d'H₂O dans le gaz à analyser, le dispositif d'analyse comprend un capteur de pression interne qui permettra d'améliorer la qualité de la mesure. En effet, l'unité laser est sensible aux variations de pression du gaz à analyser. Ainsi, en mesurant cette pression, il est possible de compenser un écart de mesure lié à la variation de pression.

Le dispositif d'analyse peut intégrer également des ressources pour le calcul de la vitesse du son du gaz, permettant corriger la mesure de la concentration d'H₂O dans le gaz à analyser.

Un autre avantage réside dans le fait que le dispositif conforme à l'invention permet, pour le biométhane, d'optimiser les temps d'analyse lesquels passent à environ 75 secondes contre environ 120 secondes pour les systèmes connus.

Le dispositif conforme à l'invention présente l'avantage qu'il ne comprend pas de sécheur d'humidité, alors que les analyseurs d'H₂O connus, comprennent un tel sécheur d'humidité, lequel nécessite une maintenance spécifique.

Le dispositif conforme à l'invention présente l'avantage qu'il ne comprend pas obligatoirement un débitmètre pour son fonctionnement, alors que la plupart des analyseurs connus intègrent un tel débitmètre. Ce dernier est effectivement indispensable dans les systèmes connus, du fait de la circulation en continu du gaz et par conséquent sont rejet continu dans l'atmosphère. Il est donc important, grâce à un débitmètre de pouvoir limiter ce débit de rejet.

En revanche, le dispositif d'analyse conforme à l'invention, avec le dimensionnement de la tubulure d'analyse et le choix d'une pression de fonctionnement, par exemple comprise entre 1 et 4 bars, avec un débit compris entre 30 ml/minute et 500 ml/minute pour tous les analyseurs intégrés au dispositif d'analyse, ainsi que le fonctionnement par intermittence, la quantité de gaz rejetée est substantiellement réduite et il est possible de s'affranchir d'un débitmètre.

Dans un autre mode de fonctionnement et de réalisation du dispositif d'analyse conforme à l'invention, une pompe est intégrée à l'unité de micro-chromatographie et un débitmètre est agencé à l'extérieur dudit dispositif. Cette pompe permet d'aspirer le gaz à analyser et le raccordement pneumatique de l'unité de micro-chromatographie en aval et en série à l'unité laser, permet d'utiliser la même pompe pour acheminer le gaz à analyser à l'unité laser ainsi qu'à l'unité de micro-chromatographie. La pompe unique est donc mutualisée. Il en résulte un énorme avantage pour le dispositif d'analyse conforme à l'invention, car aucune pompe supplémentaire et débitmètre supplémentaire ne sont nécessaires malgré l'ajout d'une unité laser.

Un autre avantage du dispositif conforme à l'invention, réside dans une exploitation des données plus simple. En effet, l'interface utilisateur, appelée aussi interface homme-machine pour l'affichage des données est unique et ne nécessite pas de licence logicielle pour son fonctionnement.

Le fait d'utiliser un seul matériel ou dispositif permet de n'avoir qu'une seule liaison de communication pour échanger des données / informations avec l'extérieur. En effet, les automates du marché qui sont raccordés en permanence sur X analyseurs pour collecter les données doivent s'adapter à chaque protocole de chaque analyseur et disposer de X liaisons de communication. Le dispositif conforme à l'invention permet d'obtenir une seule table d'échange pour l'ensemble des mesures réalisées avec une seule connexion et un protocole commun. A titre d'exemple, les protocoles connus sont Modbus ou Fieldbus, sur des supports physiques du genre RS232 ou RS485 ou Ethernet.

En outre, les modalités d'ajustage sont simplifiées puisqu'il suffit d'ajuster un seul matériel avec une seule procédure. L'interface homme machine pilote l'ensemble des mesures effectuées notamment par l'unité de micro-chromatographie et par l'unité laser et ce à l'aide d'une seule procédure et d'un seul logiciel.

L'intégration mécanique (poids, dimension, nombre de câbles électriques, tubing, etc ...) est aussi améliorée dans un dispositif conforme à l'invention.

Le dispositif d'analyse conforme à l'invention peut analyser jusqu'à 16 gaz différents sur une seule entrée.

Avantageusement, un séquencement des mesures est mis en place grâce au logiciel ou programme d'ordinateur pilotant le fonctionnement dudit dispositif d'analyse.

### Brève description des figures

D'autres caractéristiques et avantages de l'invention apparaîtront à la lecture de la description détaillée qui va suivre, description faite en référence aux dessins annexés, donnés à titre d'exemples illustratifs et non limitatifs, dans lesquels :
- la figure 1 est une illustration schématique et fonctionnelle d'un exemple de réalisation d'un dispositif d'analyse de gaz conforme à l'invention, et
- la figure 2 est une illustration d'un exemple de réalisation d'un poste d'injection de biométhane comprenant un dispositif d'analyse conforme à l'invention.

### Exposé détaillé de l'invention

Dans la suite, les éléments structurellement et fonctionnellement identiques, représentés sur des figures distinctes sont repérés par les mêmes références numériques ou alphanumériques.

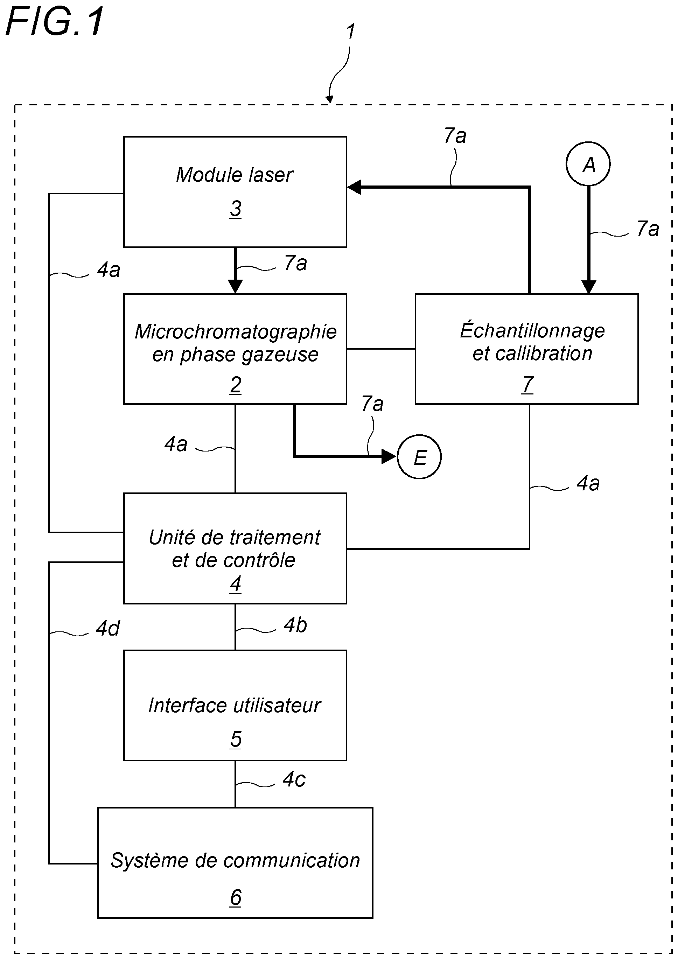

La figure 1 est une illustration schématique, pneumatique et électrique, d'un exemple de réalisation d'un dispositif d'analyse 1 de gaz conforme à l'invention. Ce dispositif d'analyse 1 comprend une unité de micro-chromatographie 2 en phase gazeuse pour mesurer la concentration de composés sous forme gazeuse.

Le dispositif d'analyse 1 comprend également une unité laser 3 comprenant au moins un module laser pour mesurer la concentration de composés complémentaires, par exemple H₂O ou NH₃.

L'unité de micro-chromatographie 2 et l'unité laser 3 sont connues, tant structurellement que fonctionnellement et ne sont donc pas décrites davantage dans la présente description.

L'une unité laser 3, comportant au moins un module laser, est raccordée en série et en amont à l'unité de micro-chromatographie 2.

Le dispositif d'analyse 1 comprend par ailleurs une unité de traitement et de contrôle 4 permettant notamment l'acquisition, le traitement, la restitution de résultats des mesures et des calculs effectués à partir de valeurs mesurées.

L'unité de traitement et de contrôle 4 est associée à une interface utilisateur 5, laquelle permet à l'utilisateur de suivre les opérations d'analyse et le cas échéant d'intervenir dans leur déroulement. L'unité de traitement et de contrôle 4 permet par exemple de réaliser divers ajustages, de paramétrer des seuils d'alarme ou de visualiser des courbes. L'unité de traitement et de contrôle 4 est reliée via une liaison filaire 4b à l'interface utilisateur 5.

Selon un exemple de réalisation du dispositif d'analyse 1, l'interface utilisateur 5 est connectée avantageusement, via une liaison filaire 4c, à un système de communication 6. Ce dernier permet notamment de transmettre des données vers un serveur de l'exploitant et de recevoir par exemple des données se rapportant à des alertes de dysfonctionnement ou de mise à jour des données d'analyse.

Selon un exemple de réalisation du dispositif d'analyse 1, l'unité de traitement et de contrôle 4 est connectée avantageusement, via une liaison filaire 4d, au système de communication 6. Ce dernier est avantageusement configuré pour échanger des données, des informations et ou instructions via le réseau internet ou autres.

Selon un autre exemple de réalisation du dispositif d'analyse 1, les liaisons filaires 4b, 4c et 4d ou un partie d'entre elles, peuvent être remplacées par liaisons de communication non filaires.

Le dispositif d'analyse 1 comprend une unité d'échantillonnage et de calibration 7 pour prélever des échantillons de gaz destinés à être analysés.

L'unité de traitement et de contrôle 4 est reliée avantageusement à l'unité laser 3, à l'unité de micro-chromatographie 2 et à l'unité d'échantillonnage et de calibration 7 par l'intermédiaire de liaisons électriques filaires 4a.

L'unité d'échantillonnage et de calibration 7 comprend une liaison pneumatique sous forme de tubulure d'analyse 7a équipée d'une d'admission de gaz A. La tubulure d'analyse 7a raccorde en série l'unité d'échantillonnage et de calibration 7, l'unité laser 3 et l'unité de micro-chromatographie 2 pour faire circuler une fraction déterminée du gaz à analyser successivement dans l'unité laser 3, puis dans l'unité de micro-chromatographie 2 avant d'être rejetée dans l'atmosphère via un évent E.

Selon un exemple de réalisation, le dispositif d'analyse 1 comprend au moins un système de prélèvement par aspiration raccordé à la tubulure d'analyse 7a. Le système de prélèvement par aspiration est avantageusement une pompe intégrée à l'unité de micro-chromatographie 2.

Selon un autre exemple de réalisation, le dispositif d'analyse 1 est dépourvu de pompe ou de système d'aspiration et fonctionne grâce à une circulation naturelle du gaz à analyser.

Avantageusement, la tubulure d'analyse 7a présente un diamètre inférieur ou égal à 3,2 mm.

Le dispositif d'analyse est également associé à un échantillonnage externe, lequel est réalisé selon les caractéristiques du gaz à analyser ainsi que selon les risques éventuels. Si par exemple le gaz à analyser présente des risques d'humidité et de surpression, un filtre anti-humidité ainsi qu'une soupape de sécurité seront disposés à l'extérieur du dispositif d'analyse 1. Une telle disposition facilite l'accessibilité et la maintenance de ces éléments. Une telle disposition permet également d'optimiser les coûts de fabrication dans la mesure où ces éléments, optionnels, ne doivent pas équiper obligatoirement le dispositif d'analyse.

De plus, le choix des éléments constitutifs du dispositif d'analyse 1, permet d'obtenir une forte diminution de la consommation de gaz. En effet :
- l'unité laser 3 ne nécessite pas de débit permanent de gaz,
- l'unité de micro-chromatographique 2 ne nécessite pas de débit permanent de gaz,
- l'ensemble de la tubulure interne d'analyse 7a est miniaturisée au maximum (diamètre inférieur à 1,6 mm) limitant les volumes qui y transitent, et
- l'absence de points froids (gaz emprisonné ou volumes morts) dans la tubulure d'analyse 7a spécifique, facilite la transmission du gaz pour l'analyse et par conséquent diminue la durée d'analyse.

En effet, des volumes emprisonnés de gaz ne sont pas représentatifs du gaz à analyser et risquent de ne pas être à la température optimale d'analyse. Ces volumes devraient alors être purgés avant de démarrer l'analyse. Avantageusement, le dispositif d'analyse 1 permet d'éviter ces inconvénients.

Le dispositif d'analyse 1 conforme à l'invention est apte à mesurer divers composés de gaz parmi lesquels on trouve de manière non exhaustive : O₂, N₂, H₂, CO, CO₂, CH₄, C₂H₆, C₃H₈, C₄H₁₀, C₅H₁₂, C₆H₁₄, C₂H₄, C₃H₆, C₄H₈, C₄H₆, C₂H₂, isomère pentane, isomère pentadiène, C₇H₁₆, C₈H₁₈, C₉H₂₀, C₁₀H₂₂, C₁₁H₂₄, C₁₂H₂₆, H₂S, COS, CH₄S, C₂H₆S, C₃H₈S, C₄H₁₀S, terbutylmercaptan, Thiophène, SO₂, CS₂, C₆H₆, C₇H₈, C₈H₁₀, C₃H₈O, CH₃Cl, Acétaldéhyde, Ethanol, Acétone, H₂O, HCN, HCL, HF, NH₃.

Les objets assignés à l'invention sont atteints également à l'aide d'un procédé d'analyse de gaz de combustion au préalable de son injection dans une canalisation ou dans un réseau de distribution, mis en oeuvre en utilisant un dispositif d'analyse présenté ci-dessus, caractérisé en ce qu'il comprend les étapes suivantes:
- ouvrir une vanne d'admission ou d'analyse pendant une durée déterminée pour faire circuler le gaz à analyser dans la tubulure d'analyse,
- prélever un échantillon de gaz par le système de prélèvement par aspiration,
- soumettre l'échantillon à l'unité laser,
- soumettre l'échantillon à l'unité de micro-chromatographie,
- effectuer un traitement et un contrôle des valeurs mesurées de manière à fournir des résultats fiables et exploitables, et
- répéter les étapes ci-dessus avec une périodicité déterminée.

Selon un exemple de mise en oeuvre du procédé d'analyse, la périodicité pour la répétition de ses étapes de mise en oeuvre est comprise entre 70 secondes et 75 secondes et la durée d'analyse est comprise entre 70 secondes et 75 secondes.

Le procédé d'analyse consiste par exemple à sélectionner, parmi un liste de composants du gaz à analyser, une partie au moins desdits composants, lesquels seront analysés. Une telle sélection permet de cibler des composants particuliers liés par exemple à la nature ou à l'origine du gaz à analyser.

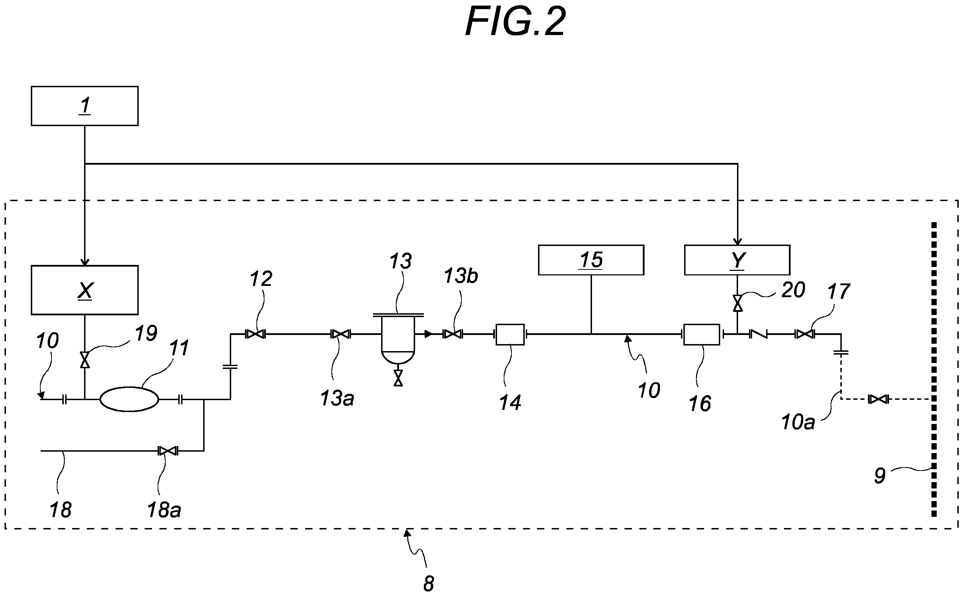

La figure 2 est une illustration d'un exemple de réalisation d'un poste d'injection 8 de bio-méthane comprenant un dispositif d'analyse 1 conforme à l'invention. Le poste d'injection 8 est destiné à injecter le gaz, en l'occurrence du bio-méthane, dans une canalisation 9. Cette dernière est par exemple intégrée dans un réseau de transport de gaz.

Le poste d'injection 8 comprend une conduite d'alimentation 10 comportant successivement un réservoir tampon 11 lequel permet de temporiser le gaz en fonction du temps d'analyse, une première vanne de coupure 12, un filtre 13, un compteur 14, un système pour l'odorisation 15, un mélangeur 16, et une portion de conduite 10a raccordée à la canalisation 9. La portion de conduite 10a comporte avantageusement au moins une vanne de coupure supplémentaire 17.

Avantageusement, la conduite d'alimentation 10 comporte deux vannes de coupure additionnelles 13a et 13b, localisées respectivement en amont et en aval du filtre 13, pour isoler ledit filtre 13 à des fins de maintenance ou de remplacement.

Le poste d'injection 8 comprend également une conduite de recyclage 18 connectée à la conduite d'alimentation 10 en aval du réservoir tampon 11 et en amont de la première vanne de coupure 12. La conduite de recyclage 18 comporte une vanne de recyclage 18a, laquelle permet, lorsqu'elle est en position ouverte, de réacheminer un gaz non conforme à la distribution, vers le producteur du gaz ou vers une autre installation prévue pour traiter et recycler ledit gaz non conforme.

Le dispositif d'analyse 1 est par exemple raccordé sur la conduite d'alimentation 10 en amont du réservoir tampon 11 via une première vanne d'analyse 19 pilotée et en aval du mélangeur 16 via un deuxième vanne d'analyse 20 pilotée.

L'ouverture de la première vanne d'analyse 19 permet ainsi de mettre en oeuvre les opérations d'analyse de la qualité du gaz, identifiées schématiquement par la lettre X. L'ouverture de la deuxième vanne d'analyse 20 permet de mettre en oeuvre les opérations de mesure du Tétrahydrothiophène (THT) du gaz, identifiées schématiquement par la lettre Y. Le fonctionnement du dispositif d'analyse 1 ainsi que l'ouverture et la fermeture des différentes vannes décrites ci-dessus, sont avantageusement, selon un exemple de mise en oeuvre, pilotés automatiquement par exemple par l'unité de traitement et de contrôle 4.

Ainsi, si le gaz n'est pas conforme aux exigences d'injection dans la canalisation 9 (réseau de distribution) suite à l'analyse identifiée en X, la vanne 12est fermée et ledit gaz est acheminé à une station de recyclage via la conduite de recyclage 18.

Si le gaz est conforme aux exigences d'injection dans la canalisation 9, la vanne 12 est ouverte est le gaz est alors odorisé. L'analyse identifiée en Y permet alors de vérifier que le gaz est correctement odorisé. Une fois cette vérification effectuée, le gaz est injecté dans la canalisation 9.

L'unité de traitement et de contrôle 4 comprend à cet effet un ordinateur ou automate sur lequel est chargé un programme dont les instructions sont aptes à piloter des opérations de mesure, de traitement et d'analyse, notamment les opérations d'échantillonnage et de calibration.

L'invention concerne ainsi un produit programme d'ordinateur comprenant des instructions de code de programme enregistrées sur un support lisible par ordinateur pour mettre en oeuvre les étapes du procédé d'analyse. Le support visible est avantageusement une carte électronique, insérée dans un ordinateur ou un automate.

L'unité de traitement et de contrôle 4 pilote avantageusement l'interface utilisateur 5 et le système de communication 6.

De manière évidente, l'invention ne se limite pas aux modes de réalisation préférentiels décrits précédemment et représentés sur les différentes figures, l'homme du métier pouvant y apporter de nombreuses modifications et imaginer d'autres variantes sans sortir ni de la portée, ni du cadre de l'invention définis par les revendications.

## Revendications

1. Dispositif d'analyse (1) de gaz de combustion comprenant :
- une unité de micro-chromatographie (2) en phase gazeuse pour mesurer la concentration de composés du gaz de combustion,
- une unité d'échantillonnage et de calibration (7) pour prélever les échantillons à analyser dans le gaz de combustion,
- une interface utilisateur (5) pour lancer et contrôler le déroulement de l'opération d'analyse et pour visionner les résultats de l'analyse à l'aide d'un outil d'affichage, et
- une unité laser (3) comportant au moins un module laser,
**caractérisé par** :
l'unité laser étant raccordée par une liaison pneumatique en série et en amont à l'unité de micro-chromatographie (2), pour mesurer la concentration de composés additionnels du gaz de combustion, une unité de traitement et de contrôle (4) automatisée pour l'acquisition et le traitement des mesures et pour effectuer des calculs nécessaires à partir des valeurs mesurées, l'unité laser (3) et l'unité de micro-chromatographie (2) effectuant les analyses respectives et successives sur les mêmes échantillons fournis grâce à l'unité d'échantillonnage et de calibrage (7) commune.

2. Dispositif d'analyse (1) selon la revendication 1, **caractérisé en ce que** l'unité d'échantillonnage et de calibrage (7) comprend une tubulure d'analyse équipée d'une vanne d'admission pilotée, ladite tubulure d'analyse raccordant en série l'unité laser (3) et l'unité de micro-chromatographie (2) pour faire circuler une fraction déterminée du gaz à analyser successivement dans l'unité laser (3) puis dans l'unité de micro-chromatographie (2).

3. Dispositif d'analyse (1) selon la revendication 2, **caractérisé en ce qu'**il comprend au moins un système de prélèvement par aspiration raccordé à la tubulure d'analyse.

4. Dispositif d'analyse (1) selon la revendication 2 ou 3, **caractérisé en ce que** la tubulure d'analyse présente un diamètre inférieur ou égal à 3,2 mm.

5. Dispositif d'analyse (1) selon l'une quelconque des revendications 1 à 4, **caractérisé en ce qu'**il comprend un système de communication (6) pour l'acquisition, la mise à disposition et la transmission de signaux mesurés et calculés et/ou de données.

6. Dispositif d'analyse (1) selon la revendication 5, **caractérisé en ce que** le système de communication (6) comprend des outils de réception et de transmission de données, configurés pour constituer un dispositif d'analyse (1) connecté.

7. Dispositif d'analyse (1) selon l'une quelconque des revendications 2 à 5, **caractérisé en ce qu'**il comprend une unité infra-rouge pour effectuer des mesures additionnelles sur le gaz circulant dans la tubulure d'analyse.

8. Dispositif d'analyse (1) selon l'une quelconque des revendications 1 à 6, **caractérisé en ce que** l'unité de traitement et de contrôle (4) automatisée comprend des cartes de communication Ethernet, une carte CPU, des cartes entrées / sorties et un afficheur tactile.

9. Poste d'injection (8) de gaz combustion dans une canalisation (9) ou dans un réseau de distribution, **caractérisé en ce qu'**il comprend un dispositif d'analyse (1) conforme à l'une quelconque des revendications 1 à 8.

10. Procédé d'analyse de gaz de combustion au préalable de son injection dans une canalisation (9) ou dans un réseau de distribution, mis en oeuvre en utilisant un dispositif d'analyse (1) conforme à l'une quelconque des revendications 1 à 8, **caractérisé en ce qu'**il comprend les étapes suivantes :
- ouvrir une vanne d'admission ou d'analyse (19) et/ou (20) pendant une durée déterminée pour faire circuler le gaz à analyser dans la tubulure d'analyse,
- prélever un échantillon de gaz grâce au système de prélèvement par aspiration ou par circulation naturelle dans une conduite,
- soumettre l'échantillon à l'unité laser (3),
- soumettre l'échantillon à l'unité de micro-chromatographie (2),
- effectuer un traitement et un contrôle des valeurs mesurées de manière à fournir des résultats fiables et exploitables, et
- répéter les étapes ci-dessus avec une périodicité déterminée.

11. Procédé d'analyse selon la revendication 10, **caractérisé en ce que** la période de mise en oeuvre des étapes dudit procédé est comprise entre 70 s et 75 s, pour du biométhane, de manière à fournir un résultat d'analyse actualisé périodiquement.

12. Procédé d'analyse selon la revendication 10 ou 11, **caractérisé en ce qu'**il consiste à l'aide de l'unité de traitement et de contrôle (4), de réajuster les valeurs mesurées par l'unité laser (3) en utilisant les valeurs mesurées par l'unité de micro-chromatographie (2).

13. Procédé d'analyse selon l'une quelconque des revendications 10 à 12, **caractérisé en ce qu'**il consiste à sélectionner, parmi un liste de composants du gaz à analyser, une partie au moins desdits composants, lesquels seront analysés.

14. Produit programme d'ordinateur comprenant des instructions de code de programme enregistrées sur un support lisible par ordinateur pour mettre en oeuvre les étapes du procédé selon l'une quelconque des revendications 10 à 13, lorsque ledit programme fonctionne sur un ordinateur d'un dispositif d'analyse (1) selon l'une des revendications 1 à 8.

## Patentansprüche

1. Vorrichtung (1) zur Analyse von Verbrennungsgasen, die Folgendes umfasst:
- eine Gas-Mikrochromatographieeinheit (2) zum Messen der Konzentration von Verbindungen des Verbrennungsgases,
- eine Probenentnahme- und Kalibriereinheit (7) zum Entnehmen der zu analysierenden Proben aus dem Verbrennungsgas,
- eine Benutzerschnittstelle (5) zum Starten und Steuern des Ablaufs des Analysevorgangs und zum Einsehen der Analyseergebnisse mit Hilfe eines Anzeigeinstruments und
- eine Lasereinheit (3), die mindestens ein Lasermodul umfasst,
**dadurch gekennzeichnet, dass**:
- die Lasereinheit über eine pneumatische Verbindung in Reihe und vorgeschaltet mit der Mikrochromatographieeinheit (2) verbunden ist, um die Konzentration zusätzlicher Verbindungen des Verbrennungsgases zu messen, eine automatisierte Verarbeitungs- und Steuereinheit (4) zum Erfassen und Verarbeiten der Messungen und zum Ausführen notwendiger Berechnungen aus den gemessenen Werten, wobei die Lasereinheit (3) und die Mikrochromatographieeinheit (2) die jeweiligen und aufeinanderfolgenden Analysen an denselben Proben ausführen, die mittels der gemeinsamen Probenentnahme- und Kalibriereinheit (7) bereitgestellt werden.

2. Analysevorrichtung (1) nach Anspruch 1, **dadurch gekennzeichnet, dass** die Probenentnahme- und Kalibriereinheit (7) einen Analyseschlauch umfasst, der mit einem vorgesteuerten Einlassventil ausgestattet ist, wobei der Analyseschlauch die Lasereinheit (3) und die Mikrochromatographieeinheit (2) in Reihe verbindet, um einen bestimmten Anteil des zu analysierenden Gases nacheinander durch die Lasereinheit (3) und dann durch die Mikrochromatographieeinheit (2) zirkulieren zu lassen.

3. Analysevorrichtung (1) nach Anspruch 2, **dadurch gekennzeichnet, dass** sie mindestens ein Ansaug-Probenentnahmesystem umfasst, das mit dem Analyseschlauch verbunden ist.

4. Analysevorrichtung (1) nach Anspruch 2 oder 3, **dadurch gekennzeichnet, dass** der Analyseschlauch einen Durchmesser von kleiner gleich 3,2 mm aufweist.

5. Analysevorrichtung (1) nach einem der Ansprüche 1 bis 4, **dadurch gekennzeichnet, dass** sie ein Kommunikationssystem (6) zum Erfassen, Bereitstellen und Übertragen von gemessenen und berechneten Signalen und/oder von Daten umfasst.

6. Analysevorrichtung (1) nach Anspruch 5, **dadurch gekennzeichnet, dass** das Kommunikationssystem (6) Datenempfangs- und - übertragungsinstrumente umfasst, die dazu konfiguriert sind, um eine verbundene Analysevorrichtung (1) zu bilden.

7. Analysevorrichtung (1) nach einem der Ansprüche 2 bis 5, **dadurch gekennzeichnet, dass** sie eine Infraroteinheit zum Ausführen zusätzlicher Messungen an dem durch den Analyseschlauch zirkulierenden Gas umfasst.

8. Analysevorrichtung (1) nach einem der Ansprüche 1 bis 6, **dadurch gekennzeichnet, dass** die automatisierte Verarbeitungs- und Steuereinheit (4) Ethernet-Kommunikationskarten, eine CPU-Karte, Ein/Ausgabekarten und eine berührungsempfindliche Anzeige umfasst.

9. Station zum Einspritzen (8) von Verbrennungsgas in eine Rohrleitung (9) oder in ein Verteilungsnetz, **dadurch gekennzeichnet, dass** sie eine Analysevorrichtung (1) gemäß einem der Ansprüche 1 bis 8 umfasst.

10. Verfahren zum Analysieren von Verbrennungsgas, bevor es in eine Rohrleitung (9) oder in ein Verteilungsnetz eingespritzt wird, durchgeführt unter Verwendung einer Analysevorrichtung (1) gemäß einem der Ansprüche 1 bis 8, **dadurch gekennzeichnet, dass** es die folgenden Schritte umfasst:
- Öffnen eines Einlass- oder Analyseventils (19) und/oder (20) für eine bestimmte Zeitdauer, um das zu analysierende Gas durch den Analyseschlauch zirkulieren zu lassen,
- Entnehmen einer Gasprobe mittels des Ansaug-Probenentnahmesystems oder durch eine natürliche Zirkulation in einer Leitung,
- Übergeben der Probe an die Lasereinheit (3),
- Übergeben der Probe an die Mikrochromatographieeinheit (2),
- Ausführen einer Verarbeitung und einer Kontrolle der gemessenen Werte, um zuverlässige und verwertbare Ergebnisse bereitzustellen, und
- Wiederholen der oben genannten Schritte mit einer bestimmten Periodizität.

11. Analyseverfahren nach Anspruch 10, **dadurch gekennzeichnet, dass** die Periode der Durchführung der Schritte des Verfahrens für Biomethan zwischen 70 s und 75 s liegt, um ein periodisch aktualisiertes Analyseergebnis bereitzustellen.

12. Analyseverfahren nach Anspruch 10 oder 11, **dadurch gekennzeichnet, dass** es darin besteht, mit Hilfe der Verarbeitungs- und Steuereinheit (4) die von der Lasereinheit (3) gemessenen Werte unter Verwendung der von der Mikrochromatographieeinheit (2) gemessenen Werte neu einzustellen.

13. Analyseverfahren nach einem der Ansprüche 10 bis 12, **dadurch gekennzeichnet, dass** es darin besteht, aus einer Liste von Komponenten des zu analysierenden Gases mindestens einen Teil der Komponenten auszuwählen, die analysiert werden.

14. Computerprogrammprodukt, das Programmcodeanweisungen umfasst, die auf einem computerlesbaren Medium gespeichert sind, um die Schritte des Verfahrens nach einem der Ansprüche 10 bis 13 durchzuführen, wenn das Programm auf einem Computer einer Analysevorrichtung (1) nach einem der Ansprüche 1 bis 8 läuft.

## Claims

1. A device for analysing (1) combustion gas, comprising:
- a micro-gas chromatography unit (2) for measuring the concentration of combustion gas compounds,
- a sampling and calibration unit (7) for taking the samples to be analysed in the combustion gas,
- a user interface (5) for launching and controlling the progress of the analysis operation and for viewing the results of the analysis using a display tool, and
- a laser unit (3) having at least one laser module,
**characterised by**:
the laser unit being connected in series via a pneumatic connection, upstream of the micro-chromatography unit (2), for measuring the concentration of additional compounds of the combustion gas, an automated processing and control unit (4) for acquiring and processing measurements and for carrying out necessary calculations based on the measured values, the laser unit (3) and the micro-chromatography unit (2) carrying out the respective and successive analyses on the same samples provided by the common sampling and calibration unit (7).

2. The analysis device (1) according to claim 1, **characterised in that** the sampling and calibration unit (7) comprises an analysis tube equipped with a pilot inlet valve, said analysis tube connecting in the series the laser unit (3) and the micro-chromatography unit (2) in order to circulate a determined fraction of the gas to be analysed successively in the laser unit (3) then in the micro-chromatography unit (2).

3. The analysis device (1) according to claim 2, **characterised in that** it comprises at least one suction collection system connected to the analysis tube.

4. The analysis device (1) according to claim 2 or 3, **characterised in that** the analysis tube has a diameter less than or equal to 3.2 mm.

5. The analysis device (1) according to any one of claims 1 to 4, **characterised in that** it comprises a communication system (6) for the acquisition, provision and transmission of measured and calculated signals and/or data.

6. The analysis device (1) according to claim 5, **characterised in that** the communication system (6) comprises tools for receiving and transmitting data, configured to constitute a connected analysis device (1) .

7. The analysis device (1) according to any one of claims 2 to 5, **characterised in that** it comprises an infrared unit for carrying out additional measurements on the gas circulating in the analysis tube.

8. The analysis device (1) according to any one of claims 1 to 6, **characterised in that** the automated processing and control unit (4) comprises Ethernet communication cards, a CPU card, input/output cards and a touch-sensitive display.

9. A combustion gas injection station (8) in a pipe (9) or in a distribution network, **characterised in that** it comprises an analysis device (1) according to any one of claims 1 to 8.

10. A method for analysing combustion gas prior to its injection into a pipe (9) or into a distribution network, implemented using an analysis device (1) according to any one of claims 1 to 8, **characterised in that** it comprises the following steps:
- opening an intake or analysis valve (19) and/or (20) for a determined period to circulate the gas to be analysed in the analysis tube,
- collecting a sample of gas using the suction collection system or by natural circulation in a pipe,
- submitting the sample to the laser unit (3),
- submitting the sample to the micro-chromatography unit (2),
- carrying out processing and control of the measured values so as to provide reliable and exploitable results, and
- repeating the above steps with a determined periodicity.

11. The analysis method according to claim 10, **characterised in that** the period of implementation of the steps of said method is between 70 s and 75 s, for biomethane, so as to provide a periodically updated analysis result.

12. The analysis method according to claim 10 or 11, **characterised in that** it consists with the processing and control unit (4) in readjusting the values measured by the laser unit (3) using the values measured by the micro-chromatography unit (2).

13. The analysis method according to any one of claims 10 to 12, **characterised in that** it consists in selecting, from a list of components of the gas to be analysed, at least one portion of said components, which will be analysed.

14. A computer program product comprising program code instructions recorded on a computer-readable medium for implementing the steps of the method according to any one of claims 10 to 13, when said program runs on a computer of an analysis device (1) according to one of claims 1 to 8.
